(19) Europäisches Patentamt
European Patent Office
Office européen des brevets

(11) Veröffentlichungsnummer: **0 129 115**
**A2**

(12) # EUROPÄISCHE PATENTANMELDUNG

(21) Anmeldenummer: 84106155.9

(22) Anmeldetag: 30.05.84

(51) Int. Cl.³: C 07 D 409/06
A 01 N 43/64

(30) Priorität: 11.06.83 DE 3321158

(43) Veröffentlichungstag der Anmeldung: 27.12.84 Patentblatt 84/52

(84) Benannte Vertragsstaaten: BE CH DE FR GB IT LI NL

(71) Anmelder: BAYER AG
Konzernverwaltung RP Patentabteilung
D-5090 Leverkusen 1 Bayerwerk(DE)

(72) Erfinder: Kraatz, Udo, Dr.
Andreasstrasse 22a
D-5090 Leverkusen 1(DE)

(72) Erfinder: Holmwood, Graham, Dr.
Krutscheider Weg 105
D-5600 Wuppertal 11(DE)

(72) Erfinder: Büchel, Karl Heinz, Prof. Dr.
Dabringhausener Strasse 42
D-5093 Burscheid(DE)

(72) Erfinder: Rosslenbroich, Hans-Jürgen, Dr.
Rembrandtstrasse 20
D-4019 Monheim(DE)

(72) Erfinder: Scheinpflug, Hans, Dr.
Am Thelenhof 15
D-5090 Leverkusen 1(DE)

(54) **Azolylmethyl-thienyl-carbinol-Derivate.**

(57) Azolylmethyl-thienyl-carbinol-Derivate der allgemeinen Formel

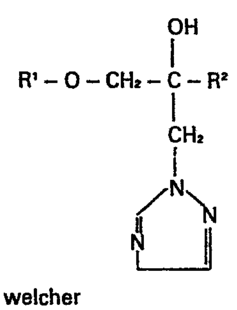

(I)

in welcher
$R^1$ für gegebenenfalls substituiertes Phenyl steht und
$R^2$ für gegebenenfalls substituiertes Thienyl steht.
Sowie ihre Verwendung als Pflanzenschutzmittel, vor allem als Fungizide.
Die neuen Azolylmethyl-thienyl-carbinol-Derivate können hergestellt werden, wenn man geeignete Oxirane mit einem geeigneten Azol umsetzt.

EP 0 129 115 A2

Croydon Printing Company Ltd

BAYER AKTIENGESELLSCHAFT 5090 Leverkusen, Bayerwerk

Konzernverwaltung RP
Patentabteilung

Slr/Bas/Kü

Ia

# Azolylmethyl-thienyl-carbinol-Derivate

Die vorliegende Erfindung betrifft neue Azolylmethyl-thienyl-carbinol-Derivate, ein Verfahren zu ihrer Herstellung und ihre Verwendung als Fungizide.

Es ist bereits bekannt geworden, daß bestimmte Hydroxy-ethyl-azolyl-Derivate, wie beispielsweise 1-(2,4-Dichlor-phenoxy)-bzw. 1-Phenoxy-3,3-dimethyl-2-(1,2,4-triazol-1-yl-methyl)-2-butanol und 1-(4-Chlorphenoxy)-2-(4-chlorphenyl)- bzw.-2-(2,4-dichlorphenyl)-3-(1,2,4-triazol-1-yl)-2-propanol, gute fungizide Eigenschaften aufweisen (vergleiche EP 0 040 345). Die Wirkung dieser Verbindungen ist jedoch nicht in allen Indikationsbereichen, insbesondere bei niedrigen Aufwandmengen bzw. -konzentrationen, immer ganz befriedigend.

Es wurden neue Azolylmethyl-thienyl-carbinol-Derivate der allgemeinen Formel

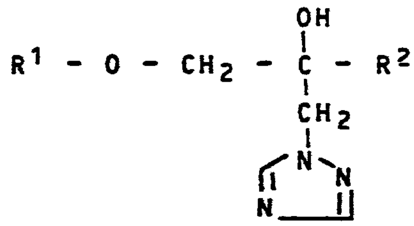

(I)

in welcher

$R^1$ für gegebenenfalls substituiertes Phenyl steht und

$R^2$ für gegebenenfalls substituiertes Thienyl steht,

gefunden.

Die Verbindungen der Formel (I) besitzen ein asymmetrisches Kohlenstoffatom und können deshalb in den beiden optischen Isomerenformen anfallen.

Weiterhin wurde gefunden, daß man die Azolylmethyl-thienyl-carbinol-Derivate der Formel (I) erhält, wenn man Oxirane der Formel

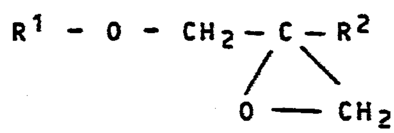

(II)

in welcher

$R^1$ für gegebenenfalls substituiertes Phenyl steht und

$R^2$ für gegebenenfalls substituiertes Thienyl steht,

mit Azolen der Formel

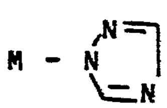

(III)

in welcher

M für Wasserstoff, Natrium, Kalium oder ein Ammoniumkation ($NR_4^{\oplus}$) steht,

in Gegenwart eines Verdünnungsmittels und gegebenenfalls in Gegenwart einer Base umsetzt.

An die so erhaltenen Verbindungen der Formel (I) kann gegebenenfalls anschließend eine Säure oder ein Metallsalz addiert werden.

Die neuen Azolylmethyl-thienyl-carbinol-Derivate der Formel (I) weisen starke fungizide Eigenschaften auf. Dabei zeigen überraschenderweise die erfindungsgemäßen Verbindungen eine bessere fungizide Wirksamkeit als die aus dem Stand der Technik bekannten Verbindungen 1-(2,4-Dichlorphenoxy)- bzw. 1-Phenoxy-3,3-dimethyl-2-(1,2,4-triazol-1-yl-methyl)-2-butanol und 1-(4-Chlorphenoxy)-2-(4-chlorphenyl)- bzw.-2-(2,4-dichlorphenyl)-3-(1,2,4-triazol-1-yl)-2-propanol, welche konstitutionell und wirkungsmäßig naheliegende Verbindungen sind. Die erfindungsgemäßen Stoffe stellen somit eine Bereicherung der Technik dar.

Außerdem sind die neuen Azolylmethyl-thienyl-carbinol-Derivate interessante Zwischenprodukte. So können z.B. die Verbindungen der allgemeinen Formel (I) an der Hydroxygruppe in üblicher Weise in die entsprechenden Ether überführt werden. Weiterhin können durch Umsetzung mit z.B. Acylhalogeniden oder Carbamoylchloriden in prinzipiell bekannter Art und Weise Acyl- oder Carbamoyl-Derivate der Verbindungen der allgemeinen Formel (I) erhalten werden.

Die erfindungsgemäßen Azolylmethyl-thienyl-carbinol-Derivate sind durch die Formel (I) allgemein definiert. In dieser Formel stehen vorzugsweise

$R^1$ für gegebenenfalls einfach bis dreifach, gleich oder verschieden substituiertes Phenyl, wobei als Substituenten genannt seien: Halogen, Alkyl mit 1 bis 4 Kohlenstoffatomen, Alkoxy und Alkylthio mit jeweils 1 oder 2 Kohlenstoffatomen; Halogenalkyl, Halogenalkoxy und Halogenalkylthio mit jeweils 1 oder 2 Kohlenstoffatomen und 1 bis 5 gleichen oder verschiedenen Halogenatomen, wie Fluor- und Chloratome, Cycloalkyl mit 5 oder 6 Kohlenstoffatomen, sowie gegebenenfalls durch Halogen substituiertes Phenyl; und

$R^2$ für gegebenenfalls einfach bis dreifach, gleich oder verschieden substituiertes Thienyl, wobei als Substituenten genannt seien: Halogen und Alkyl mit 1 bis 4 Kohlenstoffatomen.

Besonders bevorzugt sind diejenigen Verbindungen der Formel (I), in denen

$R^1$ für gegebenenfalls einfach bis zweifach, gleich oder verschieden substituiertes Phenyl steht, wobei als Substituenten genannt seien: Fluor, Chlor, Methyl, tert.-Butyl, Methoxy, Trifluormethyl, Trifluormethoxy, Phenyl und Chlorphenyl; und

$R^2$ für gegebenenfalls einfach oder zweifach, gleich oder verschieden substituiertes Thienyl steht, wobei als Substituenten genannt seien: Chlor, Brom, Methyl und Ethyl.

Bevorzugte erfindungsgemäße Verbindungen sind auch Additionsprodukte aus Säuren und denjenigen Azolylmethyl-thienyl-carbinol-Derivaten der Formel (I), in denen die Substituenten $R^1$ und $R^2$ die Bedeutungen haben, die bereits vorzugsweise für diese Substituenten genannt wurden.

Zu den Säuren, die addiert werden können, gehören vorzugsweise Halogenwasserstoffsäuren, wie z.B. die Chlorwasserstoffsäure und die Bromwasssserstoffsäure, insbesondere die Chlorwasserstoffsäure, ferner Phosphorsäure, Salpetersäure, mono- und bifunktionelle Carbonsäuren und Hydroxycarbonsäuren, wie z.B. Essigsäure, Maleinsäure, Bernsteinsäure, Fumarsäure, Weinsäure, Zitronensäure, Salizylsäure, Sorbinsäure und Milchsäure, sowie Sulfonsäuren, wie p-Toluolsulfonsäure und 1,5-Naphthalindisulfonsäure.

Außerdem bevorzugte erfindungsgemäße Verbindungen sind Additionsprodukte aus Salzen von Metallen der II. bis IV. Haupt- und der I. und II. sowie IV. bis VIII. Nebengruppe und denjenigen Azolylmethyl-thienyl-carbinol-Derivaten der Formel (I), in denen die Substituenten $R^1$ und $R^2$ die Bedeutungen haben, die bereits vorzugsweise für diese Substituenten genannt wurden.

Hierbei sind Salze des Kupfers, Zinks, Mangans, Magnesiums, Zinns, Eisens und des Nickels besonders bevorzugt. Als Anionen dieser Salze kommen solche in Betracht, die sich von solchen Säuren ableiten, die zu physiologisch verträglichen Additionsprodukten führen. Besonders bevorzugte derartige Säuren sind in diesem Zusammenhang die Halogenwasserstoffsäuren, wie z.B. die Chlorwasserstoffsäure und die Bromwasserstoffsäure, Salpetersäure und Schwefelsäure.

Verwendet man beispielsweise 2-(2-Brom-thien-5-yl)-2-(4-Chlorphenoxymethyl)-oxiran und 1,2,4-Triazol als Ausgangsstoffe, so kann der Ablauf des erfindungsgemäßen Verfahrens durch das folgende Formelschema wiedergegeben werden:

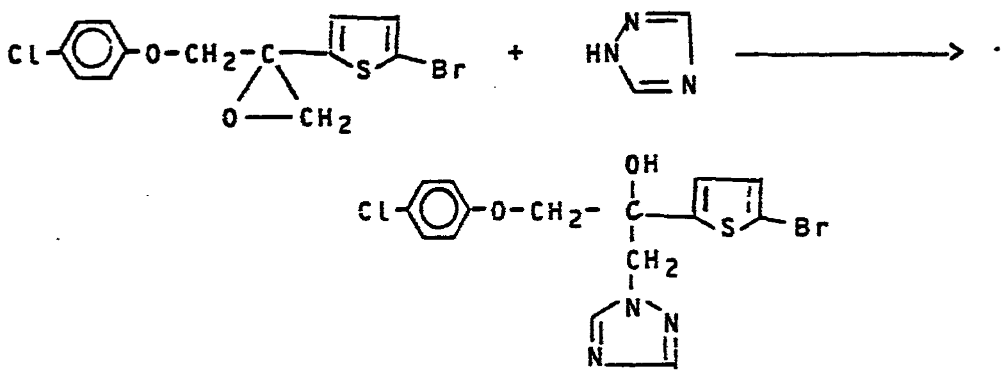

Die für die Durchführung des erfindungsgemäßen Verfahrens als Ausgangsstoffe zu verwendenden Oxirane sind durch die Formel (II) allgemein definiert. In dieser Formel haben $R^1$ und $R^2$ vorzugsweise die Bedeutungen, die bereits im Zusammenhang mit der Beschreibung der erfindungsgemäßen Stoffe der Formel (I) vorzugsweise für diese Substituenten genannt wurden.

Die Oxirane der Formel (II) sind noch nicht bekannt. Sie können jedoch in allgemein bekannter Art und Weise erhalten werden, indem man Ketone der Formel

$$R^1 - O - CH_2 - CO - R^2 \qquad (IV)$$

entweder

α) mit Dimethyloxosulfonium-methylid der Formel

$$(CH_3)_2\overset{\delta^+}{S}O\overset{\delta^-}{C}H_2 \qquad (V)$$

in an sich bekannter Weise in Gegenwart eines Verdünnungsmittels, wie beispielsweise Dimethylsulfoxid, bei Temperaturen zwischen 20 und 80°C umsetzt (vergleiche hierzu die Angaben in J.Am.Chem.Soc. 87, 1363-1364 (1965)), oder

β) mit Trimethylsulfonium-methylsulfat der Formel

$$\left[(CH_3)_3S^{(+)}\right] \quad CH_3SO_4^{(-)} \qquad (VI)$$

in an sich bekannter Weise in Gegenwart eines inerten organischen Lösungsmittels, wie z.B. Acetonitril, und in Gegenwart einer Base, wie z.B. Natriummethylat, bei Temperaturen zwischen 0 bis 60°C, vorzugsweise bei Raumtemperatur, umsetzt (vergleiche auch die Angaben in Heterocycles 8, 397 (1977)).

Die Ketone der Formel (IV) sind ebenfalls noch nicht bekannt. Auch sie können in allgemein bekannter Art und Weise erhalten werden, indem man Halogenacetyl-thiophene mit entsprechenden Phenolen in Gegenwart eines inerten organischen Lösungsmittels, wie beispielsweise Aceton, und in Gegenwart einer Base, wie beispielsweise Kaliumcarbonat, vorzugsweise unter Rückflußtemperatur umsetzt (vergleiche auch die Herstellungsbeispiele).

Als Verdünnungsmittel kommen für das erfindungsgemäße Verfahren unter den Reaktionsbedingungen inerte organische Lösungsmittel infrage. Hierzu gehören vorzugsweise Alkohole, wie z.B. Ethanol, Methoxyethanol oder Propanol; Ketone, wie z.B. 2-Butanon; Nitrile, wie z.B. Acetonitril; Ester, wie z.B. Essigester; Ether, wie z.B. Dioxan; aromatische Kohlenwasserstoffe, wie z.B. Benzol und Toluol; oder Amide, wie z.B. Dimethylformamid.

Als Basen kommen für die erfindungsgemäße Umsetzung alle üblicherweise verwendbaren anorganischen und organischen Basen in Betracht. Hierzu gehören vorzugsweise Alkalicarbonate, wie z.B. Natrium- und Kaliumcarbonat; Alkalihydroxide, wie z.B. Natriumhydroxid; Alkalialkoholate, wie z.B. Natrium- und Kalium-methylat und -ethylat; Alkalihydride, wie z.B. Natriumhydrid; sowie niedere tertiäre Alkylamine, Cycloalkylamine und Aralkylamine, wie insbesondere Triethylamin.

Die Reaktionstemperaturen können bei der Durchführung des erfindungsgemäßen Verfahrens in einem größeren Bereich variiert werden. Im allgemeinen arbeitet man bei Temperaturen zwischen 0 und 200°C, vorzugsweise zwischen 60 und 150°C.

Bei der Durchführung des erfindungsgemäßen Verfahrens setzt man vorzugsweise auf 1 Mol Oxiran der Formel (II) 1 bis 2 Mol Azol der Formel (III) und gegebenenfalls 1 bis 2 Mol Base ein; die Isolierung der Endprodukte erfolgt in allgemein üblicher Weise.

Die Säureadditionssalze der Verbindungen der Formel (I) können in einfacher Weise nach üblichen Salzbildungsmethoden, z.B. durch Lösen einer Verbindung der Formel (I) in einem geeigneten inerten Lösungsmittel und Hinzufügen der Säure, z.B. Chlorwasserstoffsäure, erhalten werden und in bekannter Weise, z.B. durch Abfiltrieren, isoliert und gegebenenfalls durch Waschen mit einem inerten organischen Lösungsmittel gereinigt werden.

Die Metallsalz-Komplexe von Verbindungen der Formel (I) können in einfacher Weise nach üblichen Verfahren erhalten werden, so z.B. durch Lösen des Metallsalzes in Alkohol, z.B. Ethanol und Hinzufügen zu Verbindungen der Formel (I). Man kann Metallsalz-Komplexe in bekannter Weise, z.B. durch Abfiltrieren, isolieren und gegebenenfalls durch Umkristallisation reinigen.

Die erfindungsgemäßen Wirkstoffe weisen eine starke mikrobizide Wirkung auf und können zur Bekämpfung von unerwünschten Mikroorganismen praktisch eingesetzt werden. Die Wirkstoffe sind für den Gebrauch als Pflanzenschutzmittel geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Als Pflanzenschutzmittel können die erfindungsgemäßen Wirkstoffe mit besonders gutem Erfolg zur Bekämpfung von Reiskrankheiten, wie Pyricularia oryzae; von Getreidekrankheiten, wie Mehltau, Rost, Cochliobolus sativus und Pyrenophora teres; sowie gegen Apfelschorf und Bohnenrost eingesetzt werden.

Hervorzuheben ist, daß die erfindungsgemäßen Stoffe nicht nur protektive Wirkung entfalten, sondern teilweise auch systemisch wirksam sind. So gelingt es, Pflanzen gegen Pilzbefall zu schützen, wenn man die Wirkstoffe über den Boden und die Wurzel oder über das Saatgut den oberirdischen Teilen der Pflanze zuführt.

Die Wirkstoffe können in die üblichen Formulierungen übergeführt werden, wie Lösungen, Emulsionen, Spritzpulver, Suspensionen, Pulver, Stäubemittel, Schäume, Pasten, lösliche Pulver, Granulate, Aerosole, Suspensions-Emulsionskonzentrate, Saatgutpuder, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä. sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und /oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen infrage: Aromaten, wie Xylol, Toluol, oder Alkyl-naphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material, wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäureester, Polyoxyethylen-Fettalkohol-ether, z.B. Alkylarylpolyglykol-ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen oder in den verschiedenen Anwendungsformen in Mischung mit anderen bekannten Wirkstoffen vorliegen, wie Fungiziden, Bakteriziden, Insektiziden, Akariziden, Nematiziden, Herbiziden, Schutzstoffen gegen Vogelfraß, Wuchsstoffen, Pflanzennährstoffen und Bodenstrukturverbesserungsmitteln.

Die Wirkstoffe können als solche, in Form ihrer Formulierungen oder der daraus durch weiteres Verdünnen bereiteten Anwendungsformen, wie gebrauchsfertige Lösungen, Emulsionen, Suspensionen, Pulver, Pasten und Granulate

angewendet werden. Die Anwendung geschieht in üblicher Weise, z.B. durch Gießen, Tauchen, Spritzen, Sprühen, Vernebeln, Verdampfen, Injizieren, Verschlämmen, Verstreichen, Stäuben, Streuen, Trockenbeizen, Feuchtbeizen, Naßbeizen, Schlämmbeizen oder Inkrustieren.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g, benötigt.

Bei Behandlung des Bodens sind Wirkstoffkonzentrationen von 0,00001 bis 0,1 Gew.-%, vorzugsweise von 0,0001 bis 0,02 Gew.-%, am Wirkungsort erforderlich.

Herstellungsbeispiele

Beispiel 1:

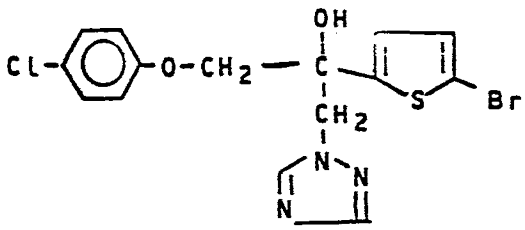

16g rohes 2-(2-Brom-thien-5-yl)-2-(4-chlorphenoxymethyl)-oxiran werden in 200 ml Acetonitril mit 11 g (0,157 Mol) 1,2,4-Triazol und 5 g Kaliumcarbonat 4 Stunden unter Rückfluß erhitzt. Danach wird das Reaktionsgemisch auf Wasser gegeben und mit Methylenchlorid extrahiert. Die Methylenchloridphase wird über Natriumsulfat getrocknet und im Vakuum eingeengt. Der Rückstand wird an Kieselgel im System Chloroform/Essigester (1:1) chromatographiert. Man erhält 10,5g (55 % der Theorie) 2-(2-Brom-thien-5-yl)-1-(4-chlorphenoxy)-3-(1,2,4-triazol-1-yl)-2-propanol in Form eines gelben Harzes, das nach langem Stehen kristallisiert. Schmelzpunkt 108-112°C.

Herstellung des Ausgangsproduktes

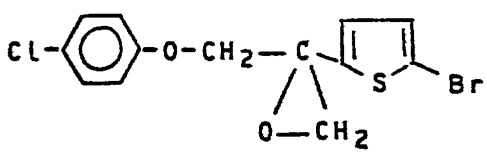

Zu einer Suspension von 11g (0,05 Mol) Trimethylsulfoxoniumiodid in 100 ml Dimethylsulfoxid gibt man unter Stickstoffatmosphäre portionsweise 1,5g (0,05 Mol) 80%-iges Natriumhydrid zu und läßt 30 Minuten bei Raumtemperatur nachrühren. Anschließend läßt man eine Lösung von 15,5g (0,046 Mol) (2-Brom-thien-5-yl)-(4-chlorphenoxymethyl)-keton in wenig Dimethylsulfoxid zutropfen und erhitzt 1 Stunde auf 60°C. Das Reaktionsgemisch wird

dann auf Wasser gegeben und mit Methylenchlorid extrahiert. Die organische Phase wird mehrmals mit Wasser gewaschen, über Magnesiumsulfat getrocknet und eingeengt. Man erhält rohes 2-(2-Brom-thien-5-yl)-2-(4-chlorphenoxymethyl)-oxiran als zähes Oel, das direkt weiter umgesetzt wird.

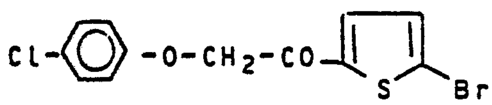

28,4g (0,1 Mol) 2-Bromacetyl-5-bromthiophen werden mit 19,3 g (0,15 Mol) 4-Chlorphenol und 27,6g (0,2 Mol) Kaliumcarbonat in 200 ml Keton unter Rühren 3 Stunden unter Rückfluß erhitzt. Man gießt dann in Wasser und extrahiert das Etherketon mit Methylenchlorid. Die organische Phase wird mit verdünnter Natronlauge und anschließend mit Wasser gewaschen und über Magnesiumsulfat getrocknet. Nach dem Eindampfen der organischen Phase wird der Rückstand aus Ethanol umkristallisiert. Man erhält 17,3 g (52 % der Theorie) (2-Brom-thien-5-yl)-(4-chlorphenoxymethyl)-keton vom Schmelzpunkt 128-130°C.

In entsprechender Weise und gemäß dem beschriebenen Verfahren werden die folgenden Verbindungen der allgemeinen Formel

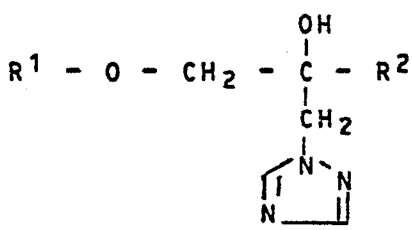

(I)

erhalten:

| Bsp. Nr. | $R^1$ | $R^2$ | Schmelzpunkt(°C) bzw. Rf-Wert(CDCl$_3$/Essigester 4 : 1) |
|---|---|---|---|
| 2 | 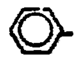 | 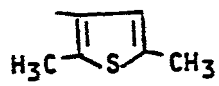 | [0,19] |
| 3 | 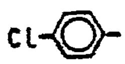 | 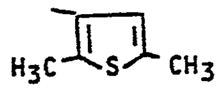 | [0,18] |
| 4 | 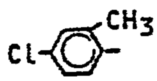 | 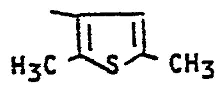 | 112-14 |
| 5 | 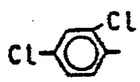 | 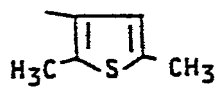 | 142 |
| 6 | 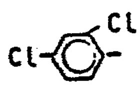 | 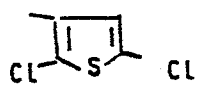 | [0,22] |

In den nachfolgenden Beispielen werden die nachstehend angegebenen Verbindungen als Vergleichssubstanzen eingesetzt:

(A) 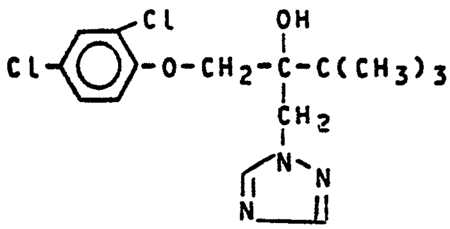

(B) 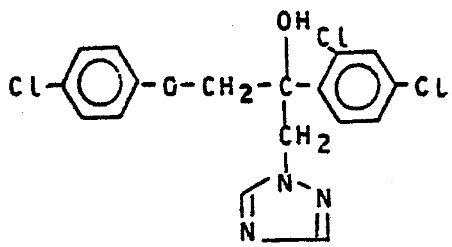

(C) 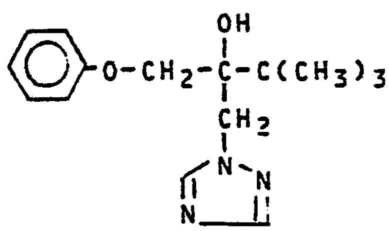

(D) 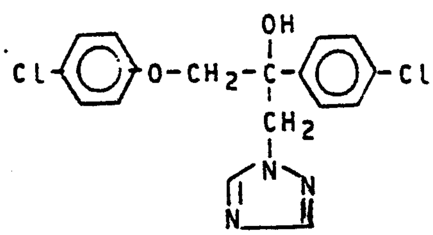

Beispiel A

Pyricularia-Test (Reis) /protektiv

Lösungsmittel: 12,5 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und verdünnt das Konzentrat mit Wasser und der angegebenen Menge Emulgator auf die gewünschte Konzentration.

Zur Prüfung auf protektive Wirksamkeit bespritzt man junge Reispflanzen mit der Wirkstoffzubereitung bis zur Tropfnässe. Nach dem Abtrocknen des Spritzbelages werden die Pflanzen mit einer wäßrigen Sporensuspension von Pyricularia oryzae inokuliert. Anschließend werden die Pflanzen in einem Gewächshaus bei 100 % rel. Luftfeuchtigkeit und 25°C aufgestellt.

4 Tage nach der Inokulation erfolgt die Auswertung des Krankheitsbefalls.

Eine deutliche Überlegenheit in der Wirksamkeit gegenüber dem Stand der Technik zeigen in diesem Test z.B. die Verbindungen gemäß folgender Herstellungsbeispiele: 1, 2, 3, 4 und 6.

Beispiel B

Pyricularia-Test (Reis) / systemisch

Lösungsmittel: 12,5 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und verdünnt das Konzentrat mit Wasser und der angegebenen Menge Emulgator auf die gewünschte Konzentration.

Zur Prüfung auf systemische Eigenschaften werden 40 ml der Wirkstoffzubereitung auf Einheitserde gegossen, in der junge Reispflanzen angezogen wurden. 7 Tage nach der Behandlung werden die Pflanzen mit einer wäßrigen Sporensuspension von Pyricularia oryzae inokuliert. Danach verbleiben die Pflanzen in einem Gewächshaus bei einer Temperatur von 25°C und einer rel. Luftfeuchtigkeit von 100 % bis zur Auswertung.

4 Tage nach der Inokulation erfolgt die Auswertung des Krankheitsbefalls.

Eine deutliche Überlegenheit in der Wirksamkeit gegenüber dem Stand der Technik zeigen in diesem Test z.B. die Verbindungen gemäß folgender Herstellungsbeispiele: 2, 3, 4, 5, 6 und 1.

Patentansprüche

1. Azolylmethyl-thienyl-carbinol-Derivate der allgemeinen Formel

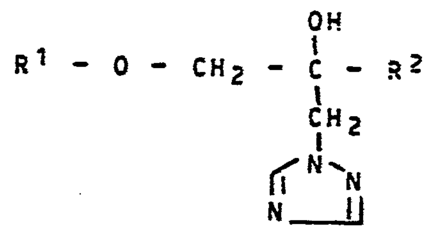

(I)

in welcher

$R^1$ für gegebenenfalls substituiertes Phenyl steht und

$R^2$ für gegebenenfalls substituiertes Thienyl steht,

sowie deren Säureadditions-Salze und Metallsalz-Kompolexe.

2. Verbindungen der Formel (I) in Anspruch 1, wobei

$R^1$ für gegebenenfalls einfach bis dreifach, gleich oder verschieden durch Halogen, Alkyl mit 1 bis 4 Kohlenstoffatomen, Alkoxy und Alkylthio mit jeweils 1 oder 2 Kohlenstoffatomen, Halogenalkyl, Halogenalkoxy und Halogenalkylthio mit jeweils 1 oder 2 Kohlenstoffatomen und 1 bis 5 gleichen oder verschiedenen Halogenatomen, Cycloalkyl mit 5 oder 6 Kohlenstoffatomen oder durch gegebenenfalls durch Halogen substituiertes Phenyl substituiertes Phenyl steht und

$R^2$ für gegebenenfalls einfach bis dreifach, gleich oder verschieden durch Halogen und Alkyl mit 1 bis 4 Kohlenstoffatomen substituiertes Thienyl steht.

3. Verbindungen der Formel (I) in Anspruch 1, wobei

$R^1$ für gegebenenfalls einfach bis zweifach, gleich oder verschieden durch Fluor, Chlor, Methyl, tert.-Butyl, Methoxy, Trifluormethyl, Trifluormethoxy, Phenyl und Chlorphenyl substituiertes Phenyl steht und

$R^2$ für gegebenenfalls einfach oder zweifach, gleich oder verschieden durch Chlor, Brom, Methyl und Ethyl substituiertes Thienyl steht.

4. Verfahren zur Herstellung von Azolylmethyl-thienyl carbinol-Derivate der allgemeinen Formel

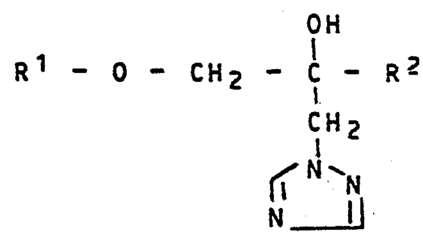

(I)

in welcher

$R^1$ für gegebenenfalls substituiertes Phenyl steht und

$R^2$ für gegebenenfalls substituiertes Thienyl steht,

dadurch gekennzeichnet, daß man Oxirane der Formel

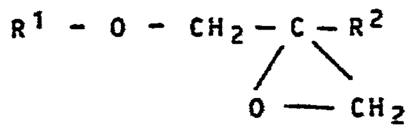

(II)

in welcher

$R^1$ und $R^2$ die oben angegebene Bedeutung haben,

mit Azolen der Formel

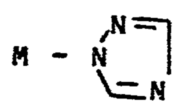

(III)

in welcher

M für Wasserstoff, Natrium, Kalium oder ein Ammoniumkation ($NR_4^{\oplus}$) steht,

in Gegenwart eines Verdünnungsmittels und gegebenenfalls in Gegenwart einer Base umsetzt und weiterhin gegebenenfalls noch an die so erhaltenen Verbindungen der Formel (I) eine Säure oder ein Metallsalz addiert.

5. Fungizide Mittel, gekennzeichnet durch einen Gehalt an mindestens einem Azolylmethyl-thienyl-carbinol-Derivat der Formel (I), gemäß Ansprüchen 1 und 4.

6. Verfahren zur Bekämpfung von Pilzen, dadurch gekennzeichnet, daß man Azolylmethyl-thienyl-carbinol-Derivate der Formel (I) gemäß Ansprüchen 1 und 4 auf Pilze oder ihren Lebensraum einwirken läßt.

7. Verwendung von Azolylmethyl-thienyl-carbinol-Derivaten der Formel (I) gemäß Ansprüchen 1 und 4 zur Bekämpfung von Pilzen.

8. Verwendung von Azolylmethyl-thienyl-carbinol-Derivaten der Formel (I) gemäß Ansprüchen 1 und 4 als Pflanzenschutzmittel.

9. Verfahren zur Herstellung von fungiziden Mitteln, dadurch gekennzeichnet, daß man Azolylmethyl-thienyl-carbinol-Derivate der Formel (I) gemäß Ansprüchen 1 und 4 mit Streckmitteln und/oder oberflächenaktiven Mitteln vermischt.